(19) Europäisches Patentamt
European Patent Office
Office européen des brevets

(11) EP 2 762 144 B1

# (12) EUROPEAN PATENT SPECIFICATION

(45) Date of publication and mention of the grant of the patent:
**12.04.2017 Bulletin 2017/15**

(21) Application number: **12836715.8**

(22) Date of filing: **26.09.2012**

(51) Int Cl.:
***A61K 31/728*** (2006.01) ***A61K 9/06*** (2006.01)
***A61K 9/08*** (2006.01) ***A61K 31/7084*** (2006.01)
***A61P 27/02*** (2006.01) ***A61P 43/00*** (2006.01)

(86) International application number:
**PCT/JP2012/074663**

(87) International publication number:
**WO 2013/047568 (04.04.2013 Gazette 2013/14)**

## (54) COMBINATION OF HYALURONIC ACID AND FLAVIN ADENINE DINUCLEOTIDE FOR USE IN INHIBITING CORNEAL EPITHELIAL CELL DEATH

KOMBINATION VON HYALURONSÄURE UND FLAVIN-ADENIN-DINUCLEOTID ZUR VERWENDUNG BEI DER HEMMUNG VON HORNHAUTEPITHELZELLTOD

COMBINAISON DE L'ACIDE HYALURONIQUE ET DE FLAVINE ADÉNINE DINUCLÉOTIDE POUR SON UTIISATION EN TANT QU'INHIBITEUR DE LA MORT CELLULAIRE ÉPITHÉLIALE DE LA CORNÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2011 JP 2011210885**

(43) Date of publication of application:
**06.08.2014 Bulletin 2014/32**

(73) Proprietor: **Santen Pharmaceutical Co., Ltd**
**Osaka-shi**
**Osaka 533-8651 (JP)**

(72) Inventors:
- **SAKAMOTO, Asuka**
  **Ikoma-shi**
  **Nara 630-0101 (JP)**
- **NAKAMURA, Masatsugu**
  **Ikoma-shi**
  **Nara 630-0101 (JP)**

(74) Representative: **Müller-Boré & Partner Patentanwälte PartG mbB**
**Friedenheimer Brücke 21**
**80639 München (DE)**

(56) References cited:
**EP-A1- 0 930 071**
**EP-A1- 1 671 629**
**EP-A1- 2 057 983**
**JP-A- 2006 117 656**

- **CHOY EMILY PIK YIN ET AL: "Investigation of corneal effect of different types of artificial tears in a simulated dry eye condition using a novel porcine dry eye model (pDEM)", CORNEA: THE JOURNAL OF CORNEA AND EXTERNAL DISEASE, LIPPINCOTT WILLIAMS & WILKINS, US, vol. 25, no. 10, 1 December 2006 (2006-12-01), pages 1200-1204, XP009182348, ISSN: 0277-3740**
- **ASUKA SAKAMOTO ET AL.: 'Effect of Flavin Adenine Dinucleotide on Ultraviolet B Induced Damage in Cultured Human Corneal Epithelial Cells' ABSTRACTS OF 132ND ANNUAL MEETING OF PHARMACEUTICAL SOCIETY OF JAPAN 3 vol. 132, no. 8, 2012, page 180, XP055127230**
- **M.NAKAMURA ET AL. FOLIA OPHTHALMOGICA JAPONICA vol. 47, no. 9, 1996, pages 1082 - 1085, XP008170638**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 762 144 B1

## Description

TECHNICAL FIELD

The present invention relates to an inhibitor for corneal epithelial cell death characterized by combining hyaluronic acid or a salt thereof (hereinafter, these are collectively called "hyaluronic acid or the like") and flavin adenine dinucleotide (FAD) or a salt thereof (hereinafter, these are collectively called "FAD or the like"), the corneal epithelial cell death being induced by ultraviolet irradiation and/or dryness. The present disclosure also relates to a method for inhibiting corneal epithelial cell death comprising administering a combination of pharmaceutically effective amounts of hyaluronic acid or the like and FAD or the like to a patient, the corneal epithelial cell death being induced by ultraviolet irradiation and/or dryness. The present invention also relates to a combination of hyaluronic acid or the like and FAD or the like for use in inhibiting corneal epithelial cell death, the corneal epithelial cell death being induced by ultraviolet irradiation and/or dryness. Further, the present invention also relates to use of a combination of hyaluronic acid or the like and FAD or the like for manufacturing an inhibitor for corneal epithelial cell death, the corneal epithelial cell death being induced by ultraviolet irradiation and/or dryness.

BACKGROUND ART

Cornea is exposed to various stresses including ultraviolet rays and dryness because it is a tissue directly contacting with the external world.

As ultraviolet rays, three kinds including ultraviolet A wave (wavelength: greater than or equal to 320 nm and less than 400 nm), ultraviolet B wave (wavelength: greater than or equal to 290 nm and less than 320 nm), and ultraviolet C wave (wavelength: greater than or equal to 200 nm and less than 290 nm) are known. Folia ophthalmologica Japonica, 57, 734-738 (2006) (Non-Patent Document 1) describes that ultraviolet B wave (hereinafter, also referred to as "UV-B") and ultraviolet C wave (hereinafter, also referred to as "UV-C") are absorbed in corneal epithelium, and that UV-B will be clinically problematic while UV-C is absorbed in the ozone layer and thus is not problematic. Further, Exp. Eye Res., 90 (2), 216-22 (2010) (Non-Patent Document 2) discloses that apoptosis (cell death) of corneal epithelial cell is induced by UV-B, and actually, Non-Patent Document 1 discloses that UV-B induces damage to corneal epithelium, corneal edema or opacity and so on.

British Journal of Ophthalmology, 85 (5), 610-612 (2001) (Non-Patent Document 3) discloses that as a result of corneal epithelial cell death induced by dryness, cells are desquamated to cause corneal ulcer and so on.

While Non-Patent Document 1 discloses that hyaluronic acid or the like inhibits increase in production of active oxygen by corneal epithelial cells and Caspase-3/7 activation by UV-B irradiation, it lacks description about whether or not hyaluronic acid or the like actually inhibits corneal epithelial cell death induced by ultraviolet irradiation. Also, Non-Patent Document 3 discloses that hyaluronic acid or the like does not inhibit cell death of corneal epithelial cells induced by dryness.

Further, regarding FAD, whether or not it actually inhibits cell death of corneal epithelial cells induced by ultraviolet irradiation has not been evidenced. While Japanese Patent Laying-Open No. 11-263729 (Patent Document 1) describes that FAD or the like has a lacrimal secretagogue action, whether or not it directly inhibits cell death of corneal epithelial cells induced by dryness is not evidenced.

As described above, whether hyaluronic acid or the like or FAD or the like inhibits corneal epithelial cell death induced by ultraviolet irradiation and/or dryness has not been evidenced, and naturally whether such an effect is achieved by a combination of hyaluronic acid or the like and FAD or the like is unclear.

While International Publication No. 2005/027893 (Patent Document 2), Japanese Patent Laying-Open No. 2005-330276 (Patent Document 3), and Japanese Patent Laying-Open No. 2005-298448 (Patent Document 4) disclose eye drops that are formulated to contain hyaluronic acid or the like and FAD or the like, whether or not the eye drops inhibit corneal epithelial cell death induced by ultraviolet irradiation and/or dryness is neither described nor suggested.

CITATION LIST

PATENT DOCUMENT

PTD 1: Japanese Patent Laying-Open No. 11-263729
PTD 2: International Publication No. 2005/027893
PTD 3: Japanese Patent Laying-Open No. 2005-330276
PTD 4: Japanese Patent Laying-Open No. 2005-298448

NON PATENT DOCUMENT

NPD 1: Folia ophthalmologica Japonica, 57, 734-738 (2006)
NPD 2: Exp. Eye Res., 90 (2), 216-22 (2010)
NPD 3: British Journal of Ophthalmology, 85 (5), 610-612 (2001)

SUMMARY OF INVENTION

TECHNICAL PROBLEM

As described above, it is an interested issue to search for drugs that inhibit corneal epithelial cell death induced by ultraviolet irradiation and/or dryness.

SOLUTION TO PROBLEM

As a result of diligent studies for searching for drugs that inhibit corneal epithelial cell death induced by ultraviolet irradiation and/or dryness, the present inventors found that a combination of hyaluronic acid or a salt thereof and flavin adenine dinucleotide or a salt thereof (hereinafter, also referred to as the "present combination") has a significant cell death inhibitory effect, and accomplished the present invention.

That is, the present invention is an inhibitor for use in corneal epithelial cell death characterized by combining hyaluronic acid or a salt thereof (hyaluronic acid or the like) and flavin adenine dinucleotide or a salt thereof (FAD or the like), the corneal epithelial cell death being induced by ultraviolet irradiation and/or dryness (hereinafter, also referred to as the "present agent").

Another aspect of the present invention is an inhibitor for use in corneal epithelial cell death characterized by combining hyaluronic acid or the like and FAD or the like, the corneal epithelial cell death being induced by ultraviolet B wave irradiation.

Still another aspect of the present invention is the present agent wherein the concentration of the hyaluronic acid or the like is 0.02 to 1% (w/v) and the concentration of the FAD or the like is 0.01 to 1% (w/v).

Still another aspect of the present invention is the present agent, wherein the concentration of the hyaluronic acid or the like is 0.02 to 0.5% (w/v) and the concentration of the FAD or the like is 0.02 to 0.5% (w/v).

Still another aspect of the present invention is the present agent, wherein the concentration of the hyaluronic acid or the like is 0.05 to 0.3% (w/v) and the concentration of the FAD or the like is 0.05% (w/v).

Still another aspect of the present invention is the present agent, wherein an administration route is ocular instillation.

Also, another aspect of the present invention is the present agent, wherein a dosage form is an ophthalmic solution.

The present disclosure also provides a method for inhibiting corneal epithelial cell death comprising administering a combination of pharmaceutically effective amounts of hyaluronic acid or the like and FAD or the like to a patient, the corneal epithelial cell death being induced by ultraviolet irradiation and/or dryness (hereinafter, also referred to as the "present method").

Another aspect of the present disclosure is a method for inhibiting corneal epithelial cell death comprising administering a combination of pharmaceutically effective amounts of hyaluronic acid or the like and FAD or the like to a patient, the corneal epithelial cell death being induced by ultraviolet B wave irradiation.

Another aspect of the present disclosure is the present method, wherein the concentration of the hyaluronic acid or the like is 0.02 to 1% (w/v) and the concentration of the FAD or the like is 0.01 to 1% (w/v).

Another aspect of the present disclosure is the present method, wherein the concentration of the hyaluronic acid or the like is 0.02 to 0.5% (w/v) and the concentration of the FAD or the like is 0.02 to 0.5% (w/v).

Another aspect of the present disclosure is the present method, wherein the concentration of the hyaluronic acid or the like is 0.05 to 0.3% (w/v) and the concentration of the FAD or the like is 0.05% (w/v).

Another aspect of the present disclosure is the present method, wherein an administration route is ocular instillation.

Also, another aspect of the present disclosure is the present method, wherein a dosage form is an ophthalmic solution.

The present invention also provides a combination of hyaluronic acid or the like and FAD or the like for use in inhibiting corneal epithelial cell death, the corneal epithelial cell death being induced by ultraviolet irradiation and/or dryness (hereinafter, also referred to as the "first present use").

Another aspect of the present invention also provides a combination of hyaluronic acid or the like and FAD or the like for use in inhibiting corneal epithelial cell death, the corneal epithelial cell death being induced by ultraviolet B wave irradiation.

Another aspect of the present invention is the first present use, wherein the concentration of the hyaluronic acid or the like is 0.02 to 1% (w/v) and the concentration of the FAD or the like is 0.01 to 1% (w/v).

Another aspect of the present invention is the first present use, wherein the concentration of the hyaluronic acid or the like is 0.02 to 0.5% (w/v) and the concentration of the FAD or the like is 0.02 to 0.5% (w/v).

Another aspect of the present invention is the first present use, wherein the concentration of the hyaluronic acid or the like is 0.05 to 0.3% (w/v) and the concentration of the FAD or the like is 0.05% (w/v).

Another aspect of the present invention is the first present use, wherein an administration route is eye drop administration.

Another aspect of the present invention is the first present use, wherein a dosage form is an ophthalmic solution.

The present invention also provides use of a combination of hyaluronic acid or the like and FAD or the like for manufacturing an inhibitor for corneal epithelial cell death, the corneal epithelial cell death being induced by ultraviolet irradiation and/or dryness (hereinafter, also referred to as the "second present use").

The present invention also provides use of a combination of hyaluronic acid or the like and FAD or the like for manufacturing an inhibitor for corneal epithelial cell death, the corneal epithelial cell death being induced by ultraviolet B wave irradiation.

Another aspect of the present invention is the second present use, wherein the concentration of the hyaluronic acid or the like is 0.02 to 1% (w/v) and the concentration of the FAD or the like is 0.01 to 1% (w/v).

Another aspect of the present invention is the second present use, wherein the concentration of the hyaluronic acid or the like is 0.02 to 0.5% (w/v) and the concentration of the FAD or the like is 0.02 to 0.5% (w/v).

Another aspect of the present invention is the second present use, wherein the concentration of the hyaluronic acid or the like is 0.05 to 0.3% (w/v) and the concentration of the FAD or the like is 0.05% (w/v).

Another aspect of the present invention is the second present use, wherein an administration route is ocular instillation.

Another aspect of the present invention is the second present use, wherein a dosage form is an ophthalmic solution.

## ADVANTAGEOUS EFFECTS OF INVENTION

As will be described later, the present combination can be an inhibitor for corneal epithelial cell death induced by ultraviolet irradiation and/or dryness because it significantly inhibits the decline of the number of living cells in corneal epithelial cells induced by ultraviolet irradiation or dryness. Also, the present disclosure provides a method for inhibiting corneal epithelial cell death comprising administering a combination of pharmaceutically effective amounts of hyaluronic acid or the like and FAD or the like to a patient, the corneal epithelial cell death being induced by ultraviolet irradiation and/or dryness. Also, the present invention provides a combination of hyaluronic acid or the like and FAD or the like for use in inhibiting corneal epithelial cell death, the corneal epithelial cell death being induced by ultraviolet irradiation and/or dryness. Also, the present invention provides use of a combination of hyaluronic acid or the like and FAD or the like for manufacturing an inhibitor for corneal epithelial cell death, the corneal epithelial cell death being induced by ultraviolet irradiation and/or dryness.

## BRIEF DESCRIPTION OF DRAWINGS

Fig. 1 is a graph showing the viability when corneal epithelial cells are treated with the present combination before UV-B irradiation (80 mJ/$cm^2$, about 1 minute).

Fig. 2 is a graph showing the viability when corneal epithelial cells are treated with the present combination after UV-B irradiation (80 mJ/$cm^2$, about 1 minute).

Fig. 3 is a graph showing the viability when corneal epithelial cells are treated with the present combination before they are exposed to dryness.

## DESCRIPTION OF EMBODIMENTS

Hyaluronic acid is a compound represented by the following general formula (1).

[Chemical formula 1]

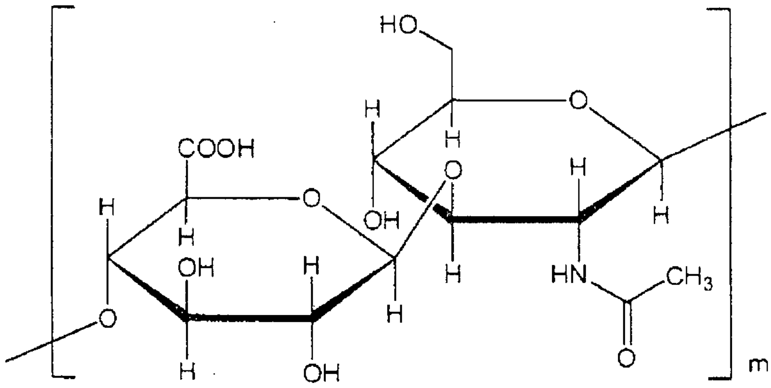

(1)

In the formula, m represents a natural number.

As the "hyaluronic acid" in the present invention, hyaluronic acid having an average molecular weight of 500,000 to 3,900,000 is preferred, and hyaluronic acid having an average molecular weight of 500,000 to 1,200,000 is more preferred.

Flavin adenine dinucleotide is a compound represented by the following formula (2).

[Chemical formula 2]

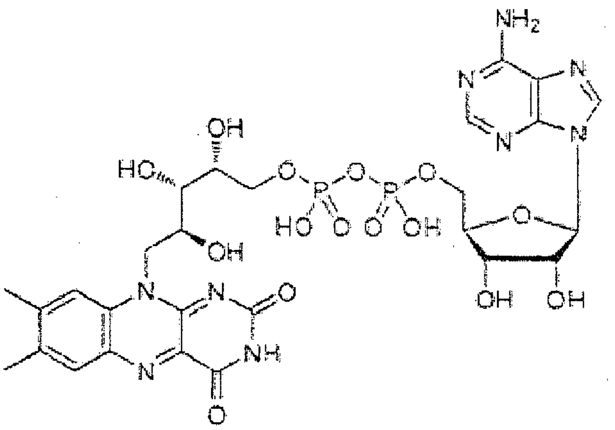

(2)

A salt of hyaluronic acid or flavin adenine dinucleotide is not particularly limited as far as it is a pharmaceutically acceptable salt, and examples include salts with an inorganic acid such as hydrochloric acid, hydrobromic acid, hydroiodic acid, nitric acid, sulfuric acid, or phosphoric acid; salts with an organic acid such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate ester, methyl sulfate, naphthalenesulfonic acid, or sulfosalicylic acid; quaternary ammonium salts with methyl bromide, methyl iodide or the like; salts with a halogen ion such as a bromine ion, a chlorine ion or an iodine ion; salts with an alkali metal such as lithium, sodium or potassium; salts with an alkaline earth metal such as calcium or magnesium; metal salts with iron, zinc or the like; salts with ammonia; and salts with an organic amine such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, or N,N-bis(phenylmethyl)-1,2-ethanediamine.

As the "salt of hyaluronic acid" in the present invention, a sodium salt is preferred, and a sodium salt represented by the following general formula (3) (hereinafter, also referred to as "sodium hyaluronate") is particularly preferred.

[Chemical formula 3]

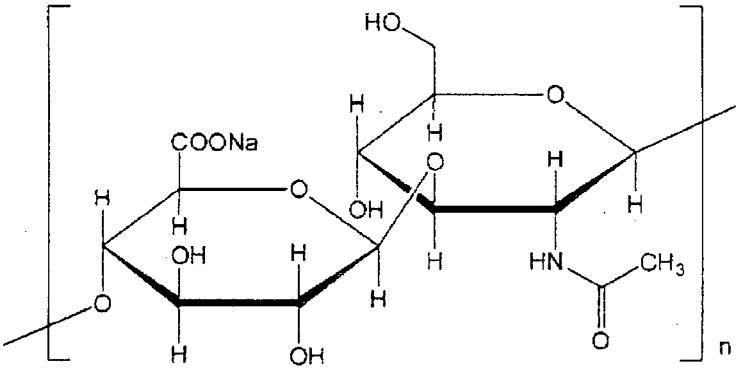

(3)

In the formula, n represents a natural number.

As the "salt of flavin adenine dinucleotide" in the present invention, a sodium salt is preferred, and a disodium salt represented by the following formula (4) (hereinafter, also referred to as "flavin adenine dinucleotide disodium salt") is particularly preferred.

[Chemical formula 4]

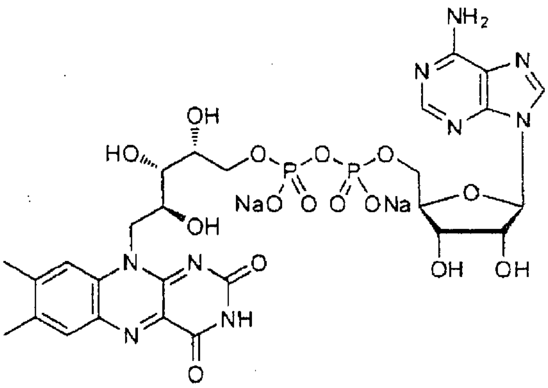

(4)

The hyaluronic acid or a salt thereof (hyaluronic acid or the like) and the flavin adenine dinucleotide or a salt thereof (FAD or the like) may be in the form of a hydrate or a solvate.

When hyaluronic acid or flavin adenine dinucleotide has geometric isomers or optical isomers, these isomers and salts thereof are also embraced in the scope of the present invention. Further, when hyaluronic acid or flavin adenine dinucleotide has proton tautomers, the tautomers or salts thereof are also embraced in the scope of the present invention.

When hyaluronic acid, flavin adenine dinucleotide or a salt thereof (including the case where it is in the form of a hydrate or solvate) has polymorphs and polymorphic group (polymorphic system), such polymorphs and polymorphic group (polymorphic system) are also embraced in the scope of the present invention. Here, the polymorphic group (polymorphic system) means individual crystalline forms in each step and the entire process in the case where the crystalline form changes with the condition and state of production, crystallization, storage and the like of the crystals (the state includes the formulated state).

Hyaluronic acid or a salt thereof and flavin adenine dinucleotide or a salt thereof may be produced according to an ordinary method in the field of organic synthetic chemistry, or a product commercially available from, for example, Sigma may be used.

In the present invention, "inhibition of corneal epithelial cell death induced by ultraviolet irradiation and/or dryness" means not only inhibiting the decline of the number of living cells in corneal epithelial cells induced by ultraviolet irradiation and/or dryness, but also means preventing or treating damage to corneal epithelium, corneal edema or opacity and so on associated with the decline of the number of living cells (namely, corneal epithelial cell death).

In the present invention, "ultraviolet rays" means the following three kinds: ultraviolet A wave (wavelength: greater than or equal to 320 nm and less than 400 nm), ultraviolet B wave (wavelength: greater than or equal to 290 nm

and less than 320 nm), and ultraviolet C wave (wavelength: greater than or equal to 200 nm and less than 290 nm). As described in the section of background art, it is known that the "ultraviolet B wave (UV-B)" is clinically problematic as it induces corneal epithelial cell death.

The present agent may be formulated into an ophthalmic agent comprising three or more kinds of active ingredients using a generally used technique by mixing other active ingredients and a pharmaceutically acceptable additive, with a combination of hyaluronic acid or a salt thereof and flavin adenine dinucleotide or a salt thereof, or may be formulated into an ophthalmic agent comprising hyaluronic acid or a salt thereof and flavin adenine dinucleotide or a salt thereof as exclusive active ingredients by using a generally used technique by adding a pharmaceutically acceptable additive to a combination of hyaluronic acid or a salt thereof and flavin adenine dinucleotide or a salt thereof.

In the present invention, the present agent is locally administered to an eye. Examples of administration route of the present agent include ocular instillation (including instillation of an ophthalmic ointment), subconjunctival administration, intra-conjunctival sac administration, and subtenon administration, with the ocular instillation being particularly preferred.

The dosage form of the present agent is not particularly limited as far as the agent is usable for local administration to an eye, and examples include an ophthalmic solution, an eye ointment, an injection solution, a patch, a gel, and an intercalating agent, with the ophthalmic solution being preferred. These can be prepared by ordinary techniques that are generally used in the field of art. Besides these preparations, the present therapeutic agent may be prepared into a preparation for intraocular implant or a preparation designed to have DDS (drug delivery system) such as a microsphere.

An ophthalmic solution can be prepared by selectively using a tonicity agent such as sodium chloride, potassium chloride, or concentrated glycerin; a buffer such as sodium phosphate, sodium acetate, or epsilon-aminocaproic acid; a surfactant such as polyoxyethylene sorbitan monooleate, polyoxyl 40 stearate, or polyoxyethylene hydrogenated castor oil; a stabilizer such as sodium citrate or sodium edetate; and a preservative such as benzalkonium chloride or paraben as is necessary. The pH of an eye drop may be within the range that is pharmaceutically acceptable for an ophthalmic preparation, and is generally preferably within the range of 4 to 8.

An ophthalmic ointment can be prepared by using a generally used base such as white vaseline or liquid paraffin.

An injection solution can be prepared by selectively using a tonicity agent such as sodium chloride; a buffer such as sodium phosphate; a surfactant such as polyoxyethylene sorbitan monooleate; and a thickener such as methylcellulose as is necessary.

An intercalating agent can be prepared by grinding and mixing a biodegradable polymer, for example, a biodegradable polymer of hydroxypropyl cellulose, hydroxypropylmethyl cellulose, carboxyvinyl polymer, polyacrylic acid or the like with the present compound, and compression molding the resultant powder, and an excipient, a binder, a stabilizer, a pH adjuster and the like may be used as is necessary.

A preparation for intraocular implant can be prepared by using a biodegradable polymer, for example, a biodegradable polymer of polylactic acid, polyglycolic acid, a lactic acid-glycolic acid copolymer, hydroxypropyl cellulose or the like.

A dose of the present agent can be varied appropriately depending on the dosage form, severity of symptom, age and body weight of the patient to which the agent is to be administered, decision by a physician and so on, and for example, when an ophthalmic solution is selected, one or two drops of the present agent containing 0.02 to 5% (w/v) of hyaluronic acid or a salt thereof and 0.005 to 5% (w/v) of flavin adenine dinucleotide or a salt thereof are instilled to an eye per one instillation, preferably one or two drops of the present agent containing 0.02 to 1% (w/v) of hyaluronic acid or a salt thereof and 0.01 to 1% (w/v) of flavin adenine dinucleotide or a salt thereof are placed per one instillation, more preferably one or two drops of the present agent comprising 0.02 to 0.5% (w/v) of hyaluronic acid or a salt thereof and 0.02 to 0.5% (w/v) of flavin adenine dinucleotide or a salt thereof are placed per one instillation, and further preferably one or two drops of the present agent comprising 0.05 to 0.3% (w/v) of hyaluronic acid or a salt thereof and 0.05% (w/v) of flavin adenine dinucleotide or a salt thereof are instilled to an eye per one instillation.

The concentrations of the hyaluronic acid or a salt thereof and the flavin adenine dinucleotide or a salt thereof can be concentrations of hyaluronic acid (free form) and flavin adenine dinucleotide (free form), and can be concentrations of salts thereof.

Dosage regimen of the present agent can be varied appropriately depending on the dosage form, severity of symptom, age and body weight of the patient to which the agent is to be administered, decision by a physician and so on, and for example, when an ophthalmic solution is selected as a dosage form, it can be locally administered to an eye in 1 to 10 divided doses daily, preferably in 2 to 8 divided doses daily, more preferably in 3 to 6 divided doses daily, and more preferably in 5 to 6 divided doses daily.

In the present invention, "characterized by combining (a) hyaluronic acid or a salt thereof, and (b) flavin adenine dinucleotide or a salt thereof" means not only administering a mixed ophthalmic agent comprising hyaluronic acid or a salt thereof and flavin adenine dinucleotide or a salt thereof at once, but also administering an ophthalmic agent comprising hyaluronic acid or a salt thereof and an ophthalmic agent comprising flavin adenine dinucleotide or a salt thereof separately and sequentially.

In Japan, for example, "Hyalein (registered trade name) ophthalmic solution 0.1%" and "Hyalein (registered trade name) ophthalmic solution 0.3%" containing only sodium hyaluronate at a concentration of 0.1% (w/v) or 0.3% (w/v) as an active ingredient, and "Flavitan (registered trade name) ophthalmic solution 0.05%" containing only flavin adenine dinucleotide disodium salt at a concentration of 0.05% (w/v) as an active ingredient are commercially available.

The present agent may be locally administered to an eye prophylactically before cornea is exposed to ultraviolet rays, and/or before cornea dries, or may be administered therapeutically after cornea is exposed to ultraviolet rays, and/or after cornea dries.

Further, the present disclosure also provides a method for inhibiting corneal epithelial cell deathcomprising administering a combination of pharmaceutically effective amounts of hyaluronic acid or the like and FAD or the like to a patient, the corneal epithelial cell death being induced by ultraviolet irradiation and/or dryness (present method), a combination of hyaluronic acid or the like and FAD or the like for use in inhibiting corneal epithelial cell death, the corneal epithelial cell death being induced by ultraviolet irradiation and/or dryness (first present use), and use of a combination of hyaluronic acid or the like and FAD or the like for manufacturing an inhibitor for corneal epithelial cell death, the corneal epithelial cell death being induced by ultraviolet irradiation and/or dryness (second present use). Definitions of terms in these aspects of the present invention are also as described above, and preferred embodiments thereof are also the same as those described above.

In the following, results of pharmacological tests and formulation examples will be shown, which are given for better understanding of the present invention but not for limiting the scope of present invention.

EXAMPLES

[Pharmacological test 1]

Whether or not the decline of the number of living cells by ultraviolet irradiation is inhibited when corneal epithelial cells are treated with the present agent before ultraviolet (UV-B) irradiation was evaluated.

(Test method)

SV40 immortalized human corneal epithelial cells (HCE-T: RIKEN BioResource Center, Japan, Cell No.: RCB2280, the same shall apply hereinafter in Examples) were inoculated in a 96-well plate ($1 \times 10^4$ cells/well), and cultured in a 10% FBS-containing DMEM/F-12 culture medium for a day. On the next day, the 10% FBS-containing DMEM/F-12 culture medium was replaced by PBS containing 0.1% (w/v) sodium hyaluronate, PBS containing 0.05% (w/v) flavin adenine dinucleotide disodium salt, PBS containing 0.1% (w/v) sodium hyaluronate and 0.05% (w/v) flavin adenine dinucleotide disodium salt, or PBS not containing a test substance (hereinafter, referred to as "0.1% HA group", "0.05% FAD group", "0.1% HA/0.05% FAD group" or "base group", respectively). Then, corneal epithelial cells were irradiated with UV-B (80 mJ/cm$^2$) for about 1 minute. After replacing the culture medium of each group by a DMEM/F-12 culture medium, the cells were cultured at 37°C four 24 hour, and then the number of living cells was determined by using Cell Titer 96 (registered trade name) AQueous One Solution Cell Proliferation Assay (product of Promega, Catalogue No.: G3580, the same shall apply hereinafter in Examples), and the viability was calculated according to the following expression 1. The sodium hyaluronate and the flavin adenine dinucleotide disodium salt used in this test were purchased from Kewpie Corporation and KYOWA HAKKO BIO CO., LTD., respectively.

[Expression 1]

Viability (%) = (Number of living cells in each group/Number of living cells in UV-B non-irradiation group) × 100

(Results)

The test results are shown in Fig. 1. In Fig.1, each value represents an average value ± standard deviation (N = 3).

(Discussion)

As is apparent from Fig. 1, when corneal epithelial cells were pre-treated with the present agent before UV-B irradiation, the viability was almost 100%. In other words, it was revealed that corneal epithelial cell death induced by ultraviolet irradiation was effectively inhibited when a combination of hyaluronic acid or a salt thereof and flavin adenine

dinucleotide or a salt thereof was locally administered to an eye prophylactically before cornea is exposed to ultraviolet rays.

[Pharmacological test 2]

A test similar to Pharmacological test 1 was conducted with the irradiation condition of UV-B varied.

(Test method)

After conducting operations similar to those in Pharmacological test 1, the 10% FBS-containing DMEM/F-12 culture medium was replaced by PBS containing 0.05% (w/v) flavin adenine dinucleotide disodium salt, PBS containing 0.05% (w/v) sodium hyaluronate, PBS containing 0.05% (w/v) sodium hyaluronate and 0.05% (w/v) flavin adenine dinucleotide disodium salt, PBS containing 0.3% (w/v) sodium hyaluronate, PBS containing 0.3% (W/V) sodium hyaluronate and 0.05% (w/v) flavin adenine dinucleotide disodium salt, or PBS not containing a test substance (hereinafter, referred to as "0.05% FAD group", "0.05% HA group", "0.05% HA/0.05% FAD group", "0.3% HA group", "0.3% HA/0.05% FAD group" or "base group", respectively). Then, corneal epithelial cells were irradiated with UV-B (120 $mJ/cm^2$) for about 2 minutes, and again operations similar to those in Pharmacological test 1 were conducted. Then, according to the expression 1, the viability was calculated, and further, according to the following expression 2, the cell death inhibition ratio of each group was calculated.

[Expression 2]

$$\text{Cell death inhibition ratio (\%)} = \text{Viability of each group} - \text{Viability of base group}$$

(Results)

The test results are shown in Table 1. In Table 1, each value represents an average value (N = 3) of cell death inhibition ratio of each group.

[Table 1]

| 0.05% FAD | 0.05% HA | 0.05% HA/0.05% FAD | 0.3% HA | 0.3% HA/0.05% FAD |
|---|---|---|---|---|
| 48.78% | -0.17% | 53.3% | 4.17% | 62.28% |

(Discussion)

As is apparent from Table 1, it was revealed that sodium hyaluronate alone can little inhibit corneal epithelial cell death by UV-B irradiation (120 $mJ/cm^2$, about 2 minutes), but can significantly enhance the cell death inhibiting effect of flavin adenine dinucleotide disodium salt when it is used in combination with flavin adenine dinucleotide sodium salt. In other words, it was revealed that when a combination of hyaluronic acid or a salt thereof and flavin adenine dinucleotide or a salt thereof is locally administered to an eye prophylactically before cornea is exposed to ultraviolet rays, these synergistically act to effectively inhibit corneal epithelial cell death induced by ultraviolet irradiation.

[Pharmacological test 3]

Whether or not the decline of the number of living cells by ultraviolet irradiation is inhibited when corneal epithelial cells are treated with the present agent after ultraviolet (UV-B) irradiation was evaluated.

(Test method)

SV40 immortalized human corneal epithelial cells were inoculated in a 96-well plate ($1 \times 10^4$ cells/well), and cultured in a 10% FBS-containing DMEM/F-12 culture medium for a day. On the next day, corneal epithelial cells were irradiated with UV-B (80 $mJ/cm^2$) for about 1 minute. Then the 10% FBS-containing DNTEM/F-1.2 culture medium was replaced by PBS containing 0.1% (w/v) sodium hyaluronate, PBS containing 0.05% (w/v) flavin adenine dinucleotide disodium salt, PBS containing 0.1% (w/v) sodium hyaluronate and 0.05% (w/v) flavin adenine dinucleotide disodium

salt, or PBS not containing a test substance, and then cultured at 37°C for 60 minutes (hereinafter, referred to as "0.1% HA group", "0.05% FAD group", "0.1% HA/0.05% FAD group" or "base group", respectively). Then, after replacing the culture medium of each group by a 10% FBS-containing DMEM/F-12 culture medium, the cells were cultured at 37°C for 24 hours, and then the number of living cells was determined by using Cell Titer 96 (registered trade name) AQueous One Solution Cell Proliferation Assay, and the viability was calculated according to the following expression 3. The sodium hyaluronate and the flavin adenine dinucleotide disodium salt used in this test were purchased from the same manufacturers as those described in Pharmacological test 1.

[Expression 3]

$$\text{Viability (\%)} = (\text{Number of living cells in each group/Number of living cells in UV-B non-irradiation group}) \times 100$$

(Results)

The test results are shown in Fig. 2. In Fig. 2, each value represents an average value ± standard deviation (N = 3).

(Discussion)

As is apparent from Fig. 2, when corneal epithelial cells were treated with sodium hyaluronate or flavin adenine dinucleotide disodium salt after UV-B irradiation, inhibition of corneal epithelial cell death was little observed. On the other hand, when corneal epithelial cells were treated with sodium hyaluronate and flavin adenine dinucleotide disodium salt, surprisingly, a significant action of inhibiting corneal epithelial cell death was observed. In other words, it was revealed that even after cornea is exposed to ultraviolet rays, corneal epithelial cell death induced by ultraviolet rays can be inhibited by local administration to an eye of a combination of hyaluronic acid or a salt thereof and flavin adenine dinucleotide or a salt thereof

[Pharmacological test 4]

Whether or not the decline of the number of living cells by dryness is inhibited when corneal epithelial cells are treated with the present agent before exposure to dryness was evaluated.

(Test method)

SV40 immortalized human corneal epithelial cells were inoculated in a 96-well plate ($1 \times 10^4$ cells/well), and cultured in a 10% FBS-containing DMEM/F-12 culture medium for a day. On the next day, the culture medium was replaced by a D-MEM/F12 culture medium containing 0.1% (w/v) sodium hyaluronate, a D-MEM/F12 culture medium containing 0.05% (w/v) flavin adenine dinucleotide disodium salt, a D-MEM/F12 culture medium containing 0.1% (w/v) sodium hyaluronate and 0.05% (w/v) flavin adenine dinucleotide disodium salt, or a D-MEM/F12 culture medium not containing a test substance, and then cells were cultured at 37°C for 60 minutes (hereinafter, referred to as "0.1% HA group", "0.05% FAD group", "0.1% HA/0.05% FAD group" or "base group", respectively). After culturing, the culture medium of each group was removed, and then cells were exposed to dryness for 15 minutes. After exposure to dryness, the number of living cells was determined by using Cell Titer 96 (registered trade name) AQueous One Solution Cell Proliferation Assay, and the viability was calculated according to the following expression 4. The sodium hyaluronate and the flavin adenine dinucleotide disodium salt used in this test were purchased from the same manufacturers as those described in Pharmacological test 1.

[Expression 4]

$$\text{Viability (\%)} = (\text{Number of living cells in each group/Number of living cells in non-dryness-exposure group}) \times 100$$

(Results)

The test results are shown in Fig. 3. In Fig. 3, each value represents an average value ± standard deviation (N = 6).

(Discussion)

As is apparent from Fig. 3, an action of inhibiting corneal epithelial cell death was observed in the 0.1% HA group, but the action was not observed in the 0.05% FAD group. On the other hand, in the 0.1% HA/0.05% FAD group, a more significant action of inhibiting corneal epithelial cell death than that of the 0.1% HA group was observed. Considering the fact that no action of inhibiting corneal epithelial cell death was observed by administration of flavin adenine dinucleotide disodium salt alone as described above, this is a surprising result. These results revealed that local administration to an eye of a combination of hyaluronic acid or a salt thereof and flavin adenine dinucleotide or a salt thereof can significantly inhibit corneal epithelial cell death by dryness.

[Formulation examples]

The drug of the present invention will be described more concretely by way of formulation examples, but it is to be noted that the present invention will not be limited to these formulation examples.

(Formulation example 1)

Ophthalmic solution (sodium hyaluronate concentration: 0.1% (w/v), flavin adenine dinucleotide disodium salt concentration: 0.05% (w/v)), in 100 ml:

| | |
|---|---|
| Sodium hyaluronate | 0.1 g |
| Flavin adenine dinucleotide disodium salt | 0.05 g |
| Sodium chloride | 0.9 g |
| Sodium hydrogen phosphate hydrate | quantum sufficit |
| Sterilized purified water | quantum sufficit |

To sterilized purified water, sodium hyaluronate, flavin adenine dinucleotide disodium salt and other ingredients as described above are added, and these are mixed sufficiently to prepare an ophthalmic solution. By varying the adding amounts of sodium hyaluronate and flavin adenine dinucleotide disodium salt, it is possible to prepare an ophthalmic solution wherein the concentration of sodium hyaluronate is 0.05% (w/v), 0.3% (w/v) or 0.5% (w/v), and the concentration of flavin adenine dinucleotide disodium salt is 0.01% (w/v) or 0.1% (w/v).

(Formulation example 2)

Ophthalmic ointment (sodium hyaluronate concentration: 0.1% (w/w), flavin adenine dinucleotide disodium salt concentration: 0.05% (w/w)), in 100g:

| | |
|---|---|
| Sodium hyaluronate | 0.1 g |
| Flavin adenine dinucleotide disodiumsalt | 0.05 g |
| Liquid paraffin | 10 g |
| White vaseline | quantum sufficit |

To white vaseline and liquid paraffin that are uniformly melted, sodium hyaluronate and flavin adenine dinucleotide disodium salt are added, and these are sufficiently mixed, and then gradually cooled to prepare an ophthalmic ointment. By varying the adding amounts of sodium hyaluronate and flavin adenine dinucleotide disodium salt, it is possible to prepare an ophthalmic ointment wherein the concentration of sodium hyaluronate is 0.05% (w/w), 0.3% (w/w) or 0.5% (w/w), and the concentration of flavin adenine dinucleotide disodium salt is 0.01% (w/w) or 0.1% (w/w).

INDUSTRIAL APPLICABILITY

A combination of hyaluronic acid or a salt thereof and flavin adenine dinucleotide or a salt thereof can be an

inhibitor for corneal epithelial cell death induced by ultraviolet irradiation and/or dryness because it significantly inhibits the decline of the number of living cells in corneal epithelial cells induced by ultraviolet irradiation or dryness.

## Claims

1. A combination of hyaluronic acid or a salt thereof and flavin adenine dinucleotide or a salt thereof for use in inhibiting corneal epithelial cell death, the corneal epithelial cell death being induced by ultraviolet irradiation and/or dryness.

2. The combination for use according to claim 1, wherein the corneal epithelial cell death is induced by ultraviolet B wave irradiation.

3. The combination for use according to claim 1 or 2, wherein the concentration of the hyaluronic acid or a salt thereof is 0.02 to 1% (w/v), and the concentration of the flavin adenine dinucleotide or a salt thereof is 0.01 to 1% (w/v).

4. The combination for use according to claim 1 or 2, wherein the concentration of the hyaluronic acid or a salt thereof is 0.02 to 0.5% (w/v), and the concentration of the flavin adenine dinucleotide or a salt thereof is 0.02 to 0.5% (w/v).

5. The combination for use according to claim 1 or 2, wherein the concentration of the hyaluronic acid or a salt thereof is 0.05 to 0.3% (w/v), and the concentration of the flavin adenine dinucleotide or a salt thereof is 0.05% (w/v).

6. The combination for use according to any one of claims 1 to 5, wherein an administration route is ocular instillation.

7. The combination for use according to claim 6, wherein a dosage form is an ophthalmic solution.

8. An inhibitor **characterized by** a combination as defined in any one of claims 1 to 7, for use in inhibiting corneal epithelial cell death being induced by ultraviolet irradiation and/or dryness.

9. The inhibitor for use according to claim 8, wherein the corneal epithelia cell death is induced by ultraviolet B wave irradiation.

## Patentansprüche

1. Kombination von Hyaluronsäure oder eines Salzes davon und Flavinadenindinukleotid oder eines Salzes davon zur Verwendung in der Hemmung des Zelltods von Corneaepithelzellen, wobei der Zelltod der Corneaepithelzellen durch ultraviolette Strahlung und/oder Trockenheit induziert ist.

2. Kombination zur Verwendung nach Anspruch 1, wobei der Zelltod der Corneaepithelzellen durch UVB-Strahlung induziert ist.

3. Kombination zur Verwendung nach Anspruch 1 oder 2, wobei die Konzentration der Hyaluronsäure oder des Salzes davon 0,02 bis 1% (w/v) und die Konzentration des Flavinadenindinukleotids oder des Salzes davon 0,01 bis 1% (w/v) ist.

4. Kombination zur Verwendung nach Anspruch 1 oder 2, wobei die Konzentration der Hyaluronsäure oder des Salzes davon 0,02 bis 0,5% (w/v) und die Konzentration des Flavinadenindinukleotids oder des Salzes davon 0,02 bis 0,5% (w/v) ist.

5. Kombination zur Verwendung nach Anspruch 1 oder 2, wobei die Konzentration der Hyaluronsäure oder des Salzes davon 0,05 bis 0,3% (w/v) und die Konzentration des Flavinadenindinukleotids oder des Salzes davon 0,05 (w/v) ist.

6. Kombination zur Verwendung nach einem der Ansprüche 1 bis 5, wobei ein Verabreichungsweg Einträufelung ins Auge ist.

7. Kombination zur Verwendung nach Anspruch 6, wobei eine Dosierungsform eine ophthalmologische Lösung ist.

8. Hemmstoff, **gekennzeichnet durch** eine Kombination wie in einem der Ansprüche 1 bis 7 definiert, zur Verwendung

in der Hemmung des Zelltods von Corneaepithelzellen, der **durch** ultraviolette Strahlung und/oder Trockenheit induziert ist.

**9.** Hemmstoff zur Verwendung nach Anspruch 8, wobei der Zelltod der Corneaepithelzellen durch UVB-Strahlung induziert ist.

## Revendications

**1.** Combinaison d'acide hyaluronique ou d'un sel de celui-ci et de flavine adénine dinucléotide ou d'un sel de celui-ci pour utilisation dans l'inhibition de la mort des cellules épithéliales de la cornée, la mort des cellules épithéliales de la cornée étant induite par un rayonnement ultraviolet et/ou par sécheresse.

**2.** Combinaison pour utilisation selon la revendication 1, où la mort des cellules épithéliales de la cornée est induite par irradiation à des ondes ultraviolettes B.

**3.** Combinaison pour utilisation selon la revendication 1 ou 2, où la concentration en acide hyaluronique ou sel de celui-ci est de 0,02 à 1 % (p/v), et la concentration en flavine adénine dinucléotide ou sel de celui-ci est de 0,01 à 1 % (p/v).

**4.** Combinaison pour utilisation selon la revendication 1 ou 2, où la concentration en acide hyaluronique ou sel de celui-ci est de 0,02 à 0,5 % (p/v), et la concentration en flavine adénine dinucléotide ou sel de celui-ci est de 0,02 à 0,5 % (p/v).

**5.** Combinaison pour utilisation selon la revendication 1 ou 2, où la concentration en acide hyaluronique ou sel de celui-ci est de 0,05 à 0,3 % (p/v), et la concentration en flavine adénine dinucléotide ou sel de celui-ci est de 0,05 % (plv).

**6.** Combinaison pour utilisation selon l'une quelconque des revendications 1 à 5, où une voie d'administration est l'instillation oculaire.

**7.** Combinaison pour utilisation selon la revendication 6, où une forme galénique est une solution ophtalmique.

**8.** Un inhibiteur **caractérisé par** une combinaison telle que définie dans l'une quelconque des revendications 1 à 7, pour utilisation dans l'inhibition de la mort des cellules épithéliales de la cornée induite par un rayonnement ultraviolet et/ou par sécheresse.

**9.** Inhibiteur pour utilisation selon la revendication 8, où la mort des cellules épithéliales de la cornée est induite par irradiation à des ondes ultraviolettes B.

FIG.1

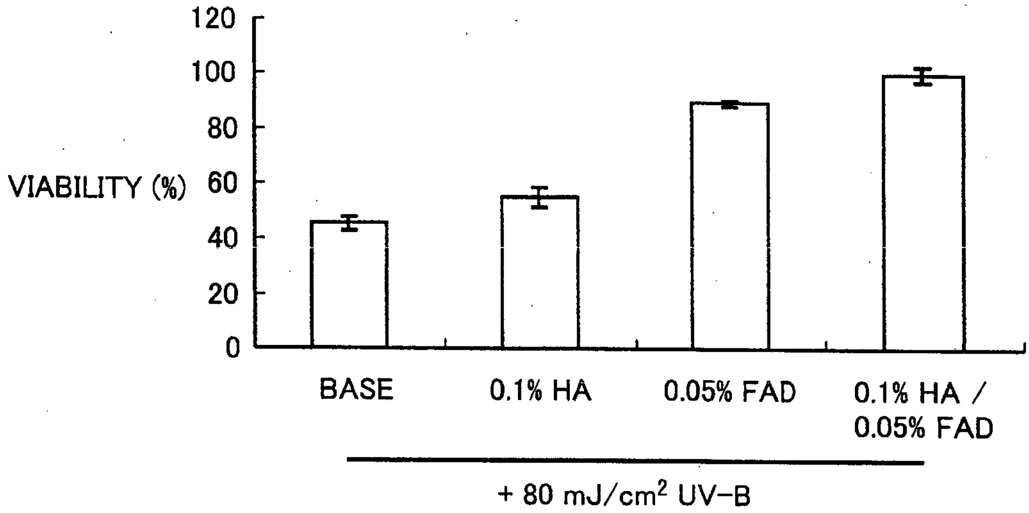
VIABILITY (%)
120
100
80
60
40
20
0
BASE
0.1% HA
0.05% FAD
0.1% HA / 0.05% FAD
+ 80 mJ/cm2 UV-B

FIG.2

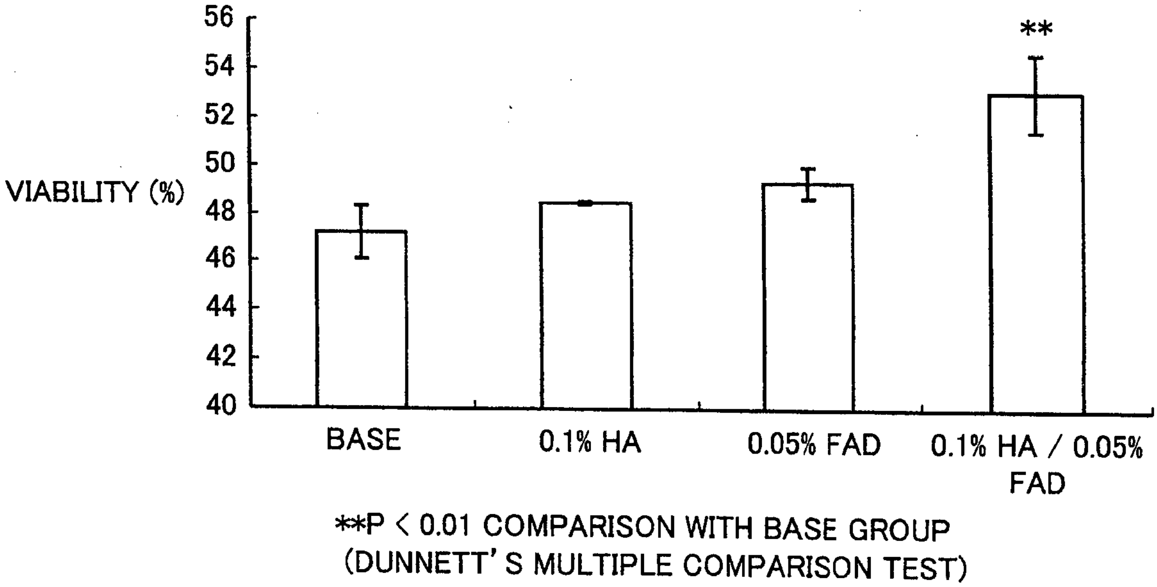
VIABILITY (%)
56
54
52
50
48
46
44
42
40
**
BASE
0.1% HA
0.05% FAD
0.1% HA / 0.05% FAD
**P < 0.01 COMPARISON WITH BASE GROUP
(DUNNETT'S MULTIPLE COMPARISON TEST)

FIG.3

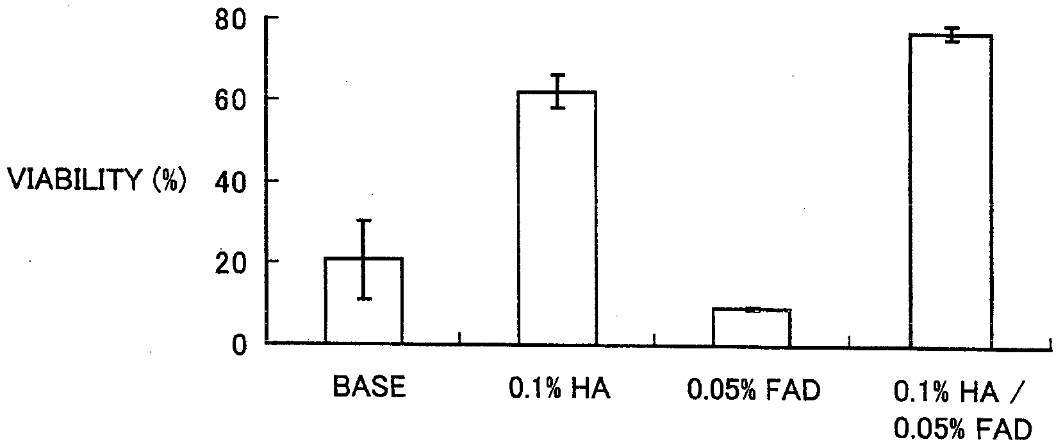

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11263729 A
- JP 2005027893 A
- JP 2005330276 A
- JP 2005298448 A


### Non-patent literature cited in the description


- *Folia ophthalmologica Japonica,* 2006, vol. 57, 734-738
- *Exp. Eye Res.,* 2010, vol. 90 (2), 216-22
- *British Journal of Ophthalmology,* 2001, vol. 85 (5), 610-612